# EUROPEAN PATENT APPLICATION

(11) **EP 3 378 380 A1**
(43) Date of publication of application: **26.09.2018**
(21) Application number: 17162379.6
(22) Date of filing: 22.03.2017
(51) Int. Cl.: A61B 5/00, A61B 5/08, A61B 5/0205, A61B 5/024

(54) **SLEEP STAGE CLASSIFICATION SYSTEM**

(71) Applicant: Koninklijke Philips N.V., 5656 AE Eindhoven (NL)
(72) Inventor: FONSECA, Pedro Miguel, 5656 AE Eindhoven (NL); RADHA, Mustafa Ghassan, 5656 AE Eindhoven (NL); AKKERMANS, Antonius Hermanus Maria, 5656 AE Eindhoven (NL)
(74) Representative: de Haan, Poul Erik

(57) **Abstract**

A sleep stage classification system (330) is provided. A sleep stage analyzing is performed based on an output signal (100a) of a PPG sensor (100). An estimation unit (310) is adapted to estimate features associated to a respiratory effort of a user based on the output signal (100a) of the PPG sensor (100) and to output a respiratory effort feature signal. A respiratory effort determining unit (320) is adapted to determine to respiratory effort based on the respiratory effort feature signal from the estimation unit (310). A sleep stage classification unit (330) is adapted to classify a sleep stage of a user based on the determined respiratory effort from the respiratory effort determining unit (320) and to output a sleep stage classification signal (310).

## Description

### FIELD OF THE INVENTION

The present invention relates to a sleep stage classification system and a method of classifying sleep stages of a user.

### BACKGROUND OF THE INVENTION

Sleep stage classification has previously been performed manually by a sleep technician. This is however considered as a very time consuming task. To improve sleep stage classification, automatic sleep stage classification has been introduced which removes the requirement for a sleep technician and allows a real time or offline sleep staging and remote monitoring of the sleep stages. Previously sleep stages have been determined by an electroencephalography. However the required sensors are considered uncomfortable. As alternative, cardiorespiratory information has been used to unobtrusively determine the sleep stages. The cardiorespiratory features required for sleep staging are determined based on electrocardiography ECG and respiratory effort signals which are measured with bands worn on the thorax of a user. This technique is, however, also obtrusive and not suitable for long term measurements.

On the other hand, optical heart rate sensors are well known to monitor or detect vital signs like a heart rate of a user. Such a heart rate sensor can be based on a photoplethysmograph (PPG) sensor and can be used to acquire a volumetric organ measurement. By means of pulse oximeters, changes in light absorption of a human skin are detected and based on these measurements a heart rate or other vital signs of a user can be determined. The PPG sensors typically comprise a light source like a light emitting diode (LED) which is emitting light into the skin of a user. The emitted light is scattered in the skin and is at least partially absorbed by the blood. Part of the light exits the skin and can be captured by a photo detector. The amount of light that is captured by the photo detector can be an indication of the blood volume inside the skin of a user. A PPG sensor can thus monitor the perfusion of blood in the dermis and subcutaneous tissue of the skin through an absorption measurement at a specific wave length. If the blood volume is changed due to the pulsating heart, the scattered light coming back from the skin of the user is also changing. Therefore, by monitoring the detected light signal in his skin by means of the photo detector, a pulse of a user and thus the heart rate can be determined. The PPG sensor can be implemented for example in a smart watch and can be placed in direct contact with the skin of the user. The PPG sensor can also be used to determine a heart rate variability HRV, for example also during longer periods of time and in a non-obtrusive manner. The heart rate variability can be determined by analyzing the distance between consecutive heart beats detected in the PPG signal.

PPG sensors have also been used however with little success in sleep stage classification.

### SUMMARY OF THE INVENTION

It is an object of the invention to provide a sleep stage classification system and a method of classifying sleep stags of a user which is reliable, unobtrusive and allows long time measurements.

According to an aspect of the invention, a sleep stage classification system is provided. The sleep stage classification system comprises a sleep stage analyzing unit which receives an output signal from a PPG sensor detecting vital signs of a user. The sleep stage analyzing unit comprises an estimation unit adapted to estimate features associated to or indicative of a respiratory effort of a user based on the output signal of the PPG sensor and to output a respiratory effort feature signal. The sleep stage analyzing unit furthermore comprises a respiratory effort determining unit adapted to determine a respiratory effort based on the respiratory effort feature signal from the estimation unit. The sleep stage analyzing unit furthermore comprises a sleep stage classification unit adapted to classify a sleep stage of a user based on the determined respiratory effort from the respiratory effort determining unit and to output a sleep stage classification signal.

The sleep stage classification is performed with an output signal of a PPG sensor as input signal.

The features associated to the respiratory effort of a user do not seem to correlate well with any reference signals from a reference measurement with a thorax belt, the features are, however, different for different sleep stages.

According to an aspect of the invention, the features associated to the respiratory effort of the user can be a pulse rate variability, a pulse amplitude variability and/or a pulse width variability.

According to a further aspect of the invention, the respiratory effort determining unit receives a predefined set of respiratory effort features and calculates a respiratory effort based on the predefined set of respiratory effort features and the respiratory effort feature signal from the estimation unit.

According to a further aspect of the invention, the sleep stage classification unit receives at least one predefined classifier and an output of the respiratory effort determining unit and classifies a sleep stage of a user. Optionally, resampling is performed.

According to a further aspect of the invention, the sleep stage classification system further comprises a heart beat detecting unit adapted to detect heart beats of a user based on the output signal of the PPG sensor. Furthermore, a body movement measuring unit is provided and is adapted to detect a movement of a user based on an output signal of a motion sensor detecting a motion of a user. Furthermore, the sleep stage classification system comprises a heart rate variability calculating unit adapted to determine a heart rate variability based on interbeat intervals detected by the heart beat detecting unit. The output of the heart rate variability calculating unit corresponds to the output of the sleep stage classification unit. The output of the body movement measuring unit is input to the sleep stage classification unit.

According to a further aspect of the invention, a method of classifying sleep stages of a user is provided. Features associated indicative of a respiratory effort of a user are estimated based on an output signal of a PPG sensor detecting vital signs of a user and a respiratory effort feature signal is output. Respiratory features are determined based on the respiratory effort feature signal. Sleep stage classification of a user is based on the determined respiratory effort features.

According to an aspect of the invention, the output data of a PPG sensor is used to estimate or determine features that are associated or indicative of a respiratory effort of a user. These estimated features are then used to determine or calculate a respiratory effort of a user. The determined or calculated respiratory effort of a user is then used in a sleep stage analyzing unit to classify a sleep stage of a user. In other words, the respiratory effort feature signal containing features associated to a respiratory effort of a user may relate to an interbeat interval (pulse rate variability), a pulse amplitude (pulse amplitude variability) and/or a pulse width (pulse width variability).

A PPG sensor according to an aspect of the invention can be implemented as a wearable device which can be for example be implemented as a wrist worn device like a smart watch. Such a PPG sensor can be implemented as a reflective PPG sensor measuring blood volume changes in the microvascular bed of tissue. Such a PPG sensor can be used to measure an average heart rate as well as measuring a heart rate variability HRV. As such a PPG sensor is implemented in a wearable device, the heart rate as well as the heart rate variability can be detected during a longer period, for example during the night period.

Regarding the heart rate variability, the distance between consecutive heart beats can be detected in the PPG signal and the heart rate variability can be calculated.

Optionally, such a PPG sensor may also comprise a motion sensor which could be implemented as an accelerometer. Accordingly, body movements as measured by the motion sensor and heart rate variability as measured by the PPG can be used to determine sleep stages as well as to determine or estimate sleep parameters.

It should be noted that the pulsatile component, namely the AC component of the PPG signal reflects heart rate activity. However, a respiratory effort is not immediately available from the PPG signal. Accordingly, without the invention, a sleeping staging performance of such PPG devices is limited as respiratory characteristics are also important when characterizing the sleep stages. However, such respiratory characteristics cannot easily be determined by the PPG signal.

According to an aspect of the invention, however, a surrogate measure of respiratory effort can be extracted from the PPG signal. Respiratory features can then be computed. Based on these computed respiratory features possibly also with detected body movements and heart rate variability characteristics from the PPG signal an automated sleep staging classification can be performed. It should be noted that the measures of the surrogate respiratory effect do not correlate well with reference respiratory effects which can be measured by a belt worn round the thorax of a user. However, the invention relates to the realization that the surrogate signal will counter-intuitively have different characteristics during different sleeping stages. Based on the different characteristics of the surrogate signal during different sleeping stages, the performance of a sleep staging classification can be improved when using the surrogate signals.

According to an aspect of the invention, the output signal of a PPG sensor is used to estimate surrogates of respiratory efforts of a user. One or more features are calculated based on this respiratory effort signal. These features can correlate with different sleeping stages or can be used to differentiate between different sleeping stages. The features determined from the respiratory effort signal can be used to perform automatic sleep stage classification based on the output signal of the PPG sensor. Optionally, the detected heart rate as well as body movements as detected by a motion sensor can be used to further improve the sleep stage classification.

It shall be understood that a preferred embodiment of the present invention can also be a combination of the dependent claims or above embodiments or aspects with respective independent claims.

These and other aspects of the invention will be apparent from and elucidated with reference to the embodiment(s) described hereinafter.

### BRIEF DESCRIPTION OF THE DRAWINGS

In the following drawings:
Fig. 1 shows a basic representation of an operational principle of a PPG sensor,
Fig. 2 shows a block diagram of sleep stage analyzing unit according to an aspect of the invention,
Fig. 3 shows a block diagram of a sleep stage analyzing unit according to a further aspect of the invention, and
Fig. 4 shows a graph indicating a pulse amplitude variability PAV, a pulse rate variability PRV and a respiratory inductance plethysmogram RIP.

### DETAILED DESCRIPTION OF EMBODIMENTS

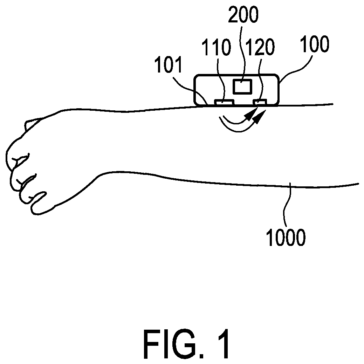

Fig. 1 shows a basic representation of an operational principle of an optical vital signs sensor. In Fig.1, the optical vital signs sensor, e.g. a heart rate sensor 100, with its contact surface 101 is arranged or placed on for example an arm of a user. The contact surface 101 can be (directly) placed onto the skin 1000 of the user. The heart rate sensor 100 comprises at least one light source 110 and at least one photo detector 120. The light source 110 emits light via the contact surface 101 onto or in the skin 1000 of a user. Some of the light is reflected and the reflected light can be detected by the photo detector 120. Some light can be transmitted through tissue of the user and be detected by the photo detector 120. Based on the reflected light, vital signs of a user like a heart rate can be determined. The optical vital signs sensor 100 can optionally comprise a motion sensor 200 which detects a motion of the arm of a user. The motion sensor 200 can for example be implemented as an acceleration sensor.

Alternatively, the PPG sensor can also be in a transmission mode, where the light is detected which is transmitted through the skin of a user.

The output signal 100a of the PPG sensor 100 gives an indication on the blood movement in vessels of a user. The quality of the output signal 100a of the PPG sensor can depend on the blood flow rate, skin morphology and skin temperature. In addition, optical losses in the PPG sensor may also have an influence on the quality of the output signal of the PPG sensor.

The PPG sensor according to an aspect of the invention can be implemented as a device that requires a contact with the skin of the user such as a wrist device (like a watch or smart watch). The optical vital signs sensor can also be implemented as a device worn behind the ear of a user, e.g. like a hearing aid or a device clamped to a finger or to an earlobe or any other body part thin enough to clip a device, and which allows light to be transmitted from one side to the other.

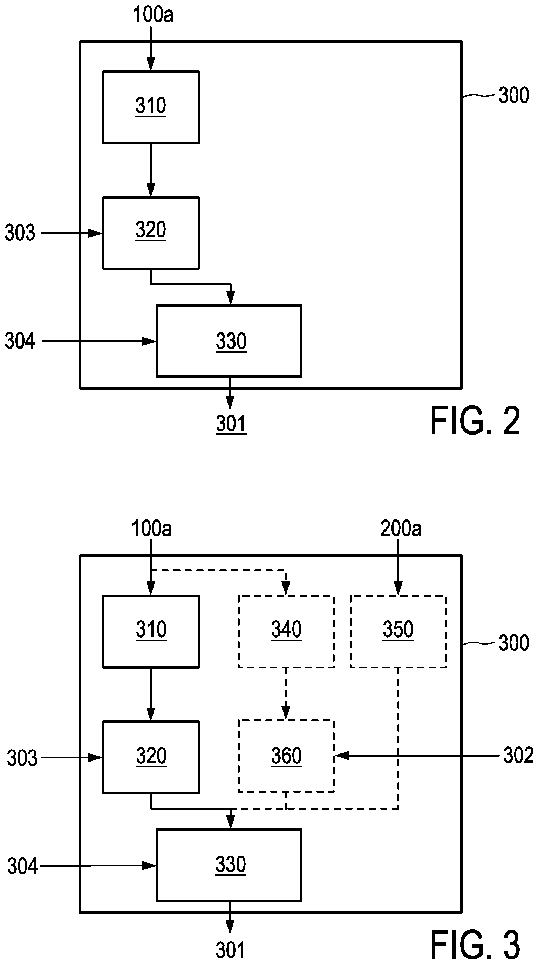

Fig. 2 shows a block diagram of a sleep stage analyzing unit according to an aspect of the invention. The sleep stage analyzing unit 300 receives output data or an output signal 100a of the PPG sensor 100. The output signal 100a of the PPG sensor 100 is input to an estimation unit 310 which estimates a surrogate of a respiratory effort. The surrogate of the respiratory effort relates to features associated to or indicative of a respiratory effort of a user. In other words, these features can relate indirectly to a respiratory effort of a user. The sleep stage analyzing unit 300 furthermore comprises a respiratory effort determining unit 320 for calculating or determining respiratory effort, in particular features which are directly related to a respiratory effort. The respiratory effort determining unit 320 may receive a pre-defined set 303 of respiratory features. The sleep stage analyzing unit 300 also comprises a sleep stage classification unit 330 which receives the output of the respiratory features determining unit 320 as well as optionally a pre-trained classifier 304. The output of the sleep stage classifying unit 330 corresponds to the classified sleep stages 301 of the user.

Fig. 3 shows a block diagram of sleep stage analyzing unit according to an aspect of the invention. The sleep staging unit 300 receives an output signal 100a of the PPG sensor 100 as input and outputs a sleep stage signal 301. The sleep stage analyzing unit 300 comprises an estimation unit 310, a respiratory effort determining unit 320 and a sleep stage classification unit 330. The sleep stage analyzing unit 300 may also receive a pre-defined set of respiratory features 303 which are input to the determining unit 320 as well as pre-trained classifier 304 which is input to the sleep stage classification unit 330.

Insofar the analyzing unit according to Fig. 3 corresponds to the analyzing unit according to Fig. 2.

In the estimating unit 310, the output signal 100a of the PPG sensor 100 is analyzed to determine or estimate a respiratory effort of a user. In particular, the estimating unit 310 serves to determine a surrogate characteristic or feature based on which sleep stages may be discriminated. A time at which a pulse p occurs corresponds to tₚ and the pulse signal corresponds to a time series Xₚ. In the estimating unit 310, the surrogates of the respiratory effort can be determined based on an inter-beat interval, a pulse amplitude and/or a pulse width.

Using the inter-beat interval, a time series corresponds to [tp] -> [argmax(Xp+1) - argmax(tp)]. This time series corresponds to the interval between adjacent peaks of the pulse of the user. The present invention considers that the time series [tp] corresponds to a respiration effort as a cardio respiratory coupling is present. The time series can also be considered as pulse rate variability PRV.

For the pulse amplitude algorithm, a time series corresponds to [tp] -> [Max(Xp) - Min(Xp)]. This algorithm is based on the consideration that the respiration of a user can induce variations in the intensity of the output signal 100a of the PPG sensor 100. It should be noted that the intensity of the output signal 100a of the PPG sensor is related or corresponds to hemodynamics such as stroke volume and blood pressure. The stroke volume and the blood pressure can be modulated by intrathoracic pressure changes which can occur at every breath because of the change of volume of the lungs. Accordingly, a pulse amplitude variability PAV can be determined.

For the pulse width algorithm, the time series corresponds to [tp] -> [|Xp|] where |Xp| is the time length of the pulse. It should be noted that the width of the pulse can capture the dispersion of blood in the catacrotic phase of the PPG output signal. This can be influenced by the pulse wave velocity. It should be noted that the pulse wave velocity is effected by blood pressure and thus can have respiratory induce modulations. Accordingly, this is also referred to pulse width variability PWV.

In the estimating unit 310, the inter-beat interval algorithm, the pulse amplitude algorithm or the pulse width algorithm or a combination of them can be performed. It should, however, be noted that the corresponding time series can be unevenly sampled. Thus, the resampling can be performed. The resampling can be performed at a fixed rate to enable further analysis (frequency analysis by means of FFT). After the resampling step, the resample signals are the output of the estimate unit 310 and correspond to the surrogate measures of respiratory effort.

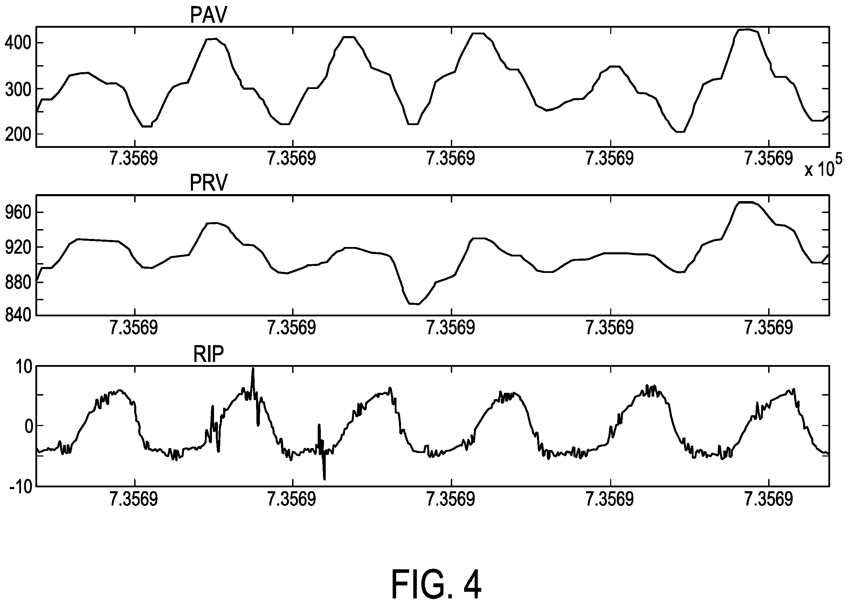

Fig. 4 shows a graph indicating a pulse amplitude variability PAV, a pulse rate variability PRV and a respiratory inductance plethysmogram RIP. In Fig. 4, the pulse amplitude variability PAV, the pulse rate variability PRV and the respiratory inductance plephysmograph RIP has been measured with a thoracic belt. Although the modulations of the pulse amplitude variability the pulse rate variability and the respiratory induced plephysmograph have similar frequency, there does not appear to be a correlation between the pulse amplitude variability, the pulse rate variability and the respiratory effort as corresponds to the RIP.

In the calculating unit 320, respiratory features are calculated or determined based on the surrogate respiratory effort (i.e. the features associated to the respiratory effort of the user) as determined by the estimating unit 310. It is known that the respiration rate and amplitude are different in different sleep stages. Accordingly, based on the respiration rate or the respiration amplitude, different sleep stages like NREM and REM can be detected. Based on the amplitude of the respiratory effort, the difference between NREM and REM can be detected in particular the amplitude of the respiratory effort is more regular during deep sleep than in other sleep stages. Although at first the surrogate measures of respiratory effort do not correlate well with any signals measured with a belt worn at the thorax, the surrogate measures of respiration are determined by the estimation unit 310 are different for different sleep stages.

According to an aspect of the invention, cardiac features can be used to discriminate between different sleep stages. As an example statistics may be determined over R-R intervals or on beats detected from a PPG sensor. Cardiac features may be calculated such that a number of intervals per epoch which relate to the average heart rate in the epoch, namely the nth percentile, a standard deviation can be calculated as well as a range of the interval length. Additional characteristics resulting from a PSD analysis can be computed over three different frequency bands namely very low frequency VLF (0,005 - 0,04 Hz), low frequency (LF): 0,04 - 0,15 Hz and high frequency (HF: 0,15 - 0,45 Hz). In addition, the modulus and the phase in the pole in the high frequency band can be calculated. Moreover, the regularity of the signal over different time scales is monitored. Detrended fluctuation analysis DFA can be used to identify long term correlations in a signal and sample entropy to quantify the self-similarity of the signal over a given time interval.

Moreover, cardiorespiratory coupling features may be used. In particular the features that may describe the cardiorespiratory coupling are the phase synchronization between R-R intervals from ECG or beats from the PPG signal and the respiratory phase measured from RIP or from PPG during a number of breathing cycles. Accordingly, based on the beats from the PPG signal or the RIP or from the output signal of the PPG sensor during a number of breathing cycles, a cardiorespiratory coupling may be determined and thus based on this sleeping stage may be estimated or determined.

According to the invention, the calculating unit 320 may receive a predetermined set of respiratory features. This feature selection may be performed separately during a training time period, wherein different sleep stages on a set of example recordings are annotated together with manual scoring based on gold standard reference PSG may be used. The predefined list may be determined by hand or may be selected based on an algorithm such as correlation feature selection.

The sleep stage classification unit 330 uses the output of the determining unit and may use trained classifiers to determine or calculate a sleep stage of a user.

It should be noted that the function of the estimation unit 310, the determining unit 320 and the sleep stage classification unit 330 is the same for the embodiments of Fig. 2 and 3. In the embodiment of Fig. 3, additionally a heart beat detecting unit 340, a body movement measuring unit 350 and a heart rate variability calculation unit 360 are present in the analyzing unit 300. The output of the body movement measuring unit 350 and the output of the heart rate variability calculation unit 360 are also input to the sleep stage classification unit 330 to enable an improved sleep stage classification. According to the invention, heart rate variability characteristics measured from the distance between consecutive beats in the PPG signal, respiratory characteristics measured by surrogate respiratory effect as determined by the estimation unit 310 based on the output of the PPG signal which was computed based on the amplitude variability algorithm and body movements determined by the motion sensor in the PPG sensor can be used.

The pre-trained classifiers 340 which are input to the sleep stage classification unit 330 can be calculated in four classes (wake (N1+N2/N3/REM) and three classes (wake/non-REM/REM) task in terms of Cohen's kappa coefficient of agreement and accuracy. These classifiers are compared with earlier recordings with a classifier using only cardiac and body movement features.

Table 1 discloses a performance comparison between a classifier trained with a heart rate variability and body movements and the classifier according to the invention based on respiratory and heart rate variability features together with body movement.

**Table 1**

| | HRV+ACT (N=54) | | RESP+HRV+ACT (N=99) | |
|---|---|---|---|---|
| | Accuracy | Kappa | Accuracy | kappa |
| 4 classes (W/R/N1+N2/N3) | 59.4 ± 8.6 | 0.42 ± 0.12 | 72.7 +/- 8.1 | 0.60 +/- 0.12 |
| 3 classes (W/R/NR) | 72.8 ± 8.7 | 0.47 ± 0.15 | 84.2 +/- 6.8 | 0.67 +/- 0.13 |

As can be seen from the table, the classifiers according to the invention enable an improved performance for classifying sleep stages.

According to an aspect of the invention, the features related to sleep-stage related respiratory information are extracted from the output signal of the PPG sensor.

They can be roughly grouped in different categories, describing different aspects of respiratory information (changes in the amplitude, changes in the respiratory frequency and features related to the spectral characteristics of respiratory information.

In the following, a "breathing cycle" is considered as the segment of the surrogate respiratory signal between two consecutive peaks in that same signal.

Changes in the amplitude can be described by: a variance of the surrogate respiratory effort signal over windows of 30, 60, 90 seconds; mean and standard deviation of signal correlations between consecutive breathing cycles; mean, median, amplitude of peaks and troughs in the surrogate respiratory signal, and median of the consecutive peak-to-trough differences; approximate entropy of peaks and troughs in the surrogate respiratory signal, expressing regularity of the sequences of peaks and troughs; sample entropy of surrogate respiratory signal over 210 seconds; area under the breathing cycle signal, under the inhalation (considering between a trough and the next peak) and under the exhalation (between a peak and the next trough) segments of the breathing cycle signal; similarity between the peaks and troughs by means of the envelope morphology using a dynamic time warping (DTW) metric and/or DTW distance (expressing dissimilarity) between neighboring segments of the surrogate respiration interval.

Changes in respiratory frequency are described by: standard deviation of the respiratory frequency over 150, 210, and 270 seconds; standard deviation of the length of consecutive breathing cycles and/or length of inhalation (time between a trough and the next peak) and exhalation (time between a peak and the next trough).

Spectral characteristics of respiratory information are described by: power of the peak in the power spectral density estimate of the surrogate respiratory signal and/or spectral power in three bands (0.01-0.05, Hz, 0.05-0.15 Hz, and 0.15-0.5 Hz) bands and ratio between the power in the second and the third bands.

These features are useful for sleep staging since they vary throughout the night depending on which sleep stage the subject is in. So any combination of one or more of these features can be used according to an aspect of the invention.

According to an aspect of the invention the output signal of a PPG sensor is analyzed to identify features that are indicative of a respiratory effort of a user.

According to an aspect of the invention features extracted from the surrogate respiratory effort are used. However, the surrogate estimate of respiratory effort correlates very poorly with actual respiratory effort (measured with standard respiratory sensors). It should be noted, that the respiratory features, despite this poor surrogate estimation, still retain their discriminatory power with respect to sleep stages.

Other variations of the disclosed embodiment can be understood and effected by those skilled in the art in practicing the claimed invention from a study of the drawings, the disclosure and the appended claims.

In the claims, the word "comprising" does not exclude other elements or steps and in the indefinite article "a" or "an" does not exclude a plurality.

A single unit or device may fulfil the functions of several items recited in the claims. The mere fact that certain measures are recited in mutual different dependent claims does not indicate that a combination of these measurements cannot be used to advantage. A computer program may be stored/distributed on a suitable medium such as an optical storage medium or a solid state medium, supplied together with or as a part of other hardware, but may also be distributed in other forms such as via the internet or other wired or wireless telecommunication systems.

Any reference signs in the claims should not be construed as limiting the scope.

## Claims

1. Sleep stage classification system, comprising:
a sleep stage analyzing unit (300) which receives an output signal (100a) of a photoplethysmograph PPG sensor (100) detecting vital signs of a user,
wherein the sleep stage analyzing unit (300) comprises an estimation unit (310) adapted to estimate features associated to a respiratory effort of a user based on the output signal (100a) of the PPG sensor (100) and to output a respiratory effort feature signal,
wherein the sleep stage analyzing unit (300) furthermore comprises a respiratory effort determining unit (320) adapted to determine a respiratory effort based on the respiratory effort feature signal from the estimation unit (310),
wherein the sleep stage analyzing unit (300) further comprises a sleep stage classification unit (330) adapted to classify a sleep stage of a user based on the determined respiratory effort from the respiratory effort determining unit (320), and to output a sleep stage classification signal (301).

2. Sleep stage classification system, wherein
the features associated to a respiratory effort of a user or the respiratory effort feature signal comprise a pulse rate variability, a pulse amplitude variability and/or a pulse width variability.

3. Sleep stage classification system according to claim 1, wherein
the respiratory effort determining unit (320) receives a predefined set of respiratory effort features and calculates respiratory effort based on the predefined set of respiratory effort and the respiratory effort feature signal from the estimation unit (310).

4. Sleep stage classification system according to claim 3, wherein
the sleep stage classification unit (330) receives at least one pre-trained classifier (340) and an output of the respiratory effort determining unit (320) and classifies a sleep stage of a user.

5. Sleep stage classification system according to claim 3, further comprising:
a heart beats detecting unit (340) adapted to detect heart beats of a user based on the output signal (300a) of the PPG sensor (100),
a body movement measuring unit (350) adapted to detect a movement of a user based on output signal (200a) of a motion sensor (200) detecting a motion of a user, and
a heart rate variability calculation unit (360) adapted to determine a heart rate variability based on interbeat intervals detected by the heart beat detection unit (340), wherein the output of the heart rate variability calculation unit (360) corresponds to the output of the sleep stage classification unit (330),
wherein the output of the body movement measuring unit (130) is input to the sleep stage classification unit (330).

6. Method of classifying sleep stages of a user,
estimating features associated to respiratory efforts based on an output signal (100a) of a PPG sensor (100) detecting vital signs of a user and outputting respiratory effort feature signal,
determining respiratory effort features based on respiratory effort feature signal, and
sleep stage classifying of a user based on the determined respiratory effort features.
